# FASCICULE DE BREVET EUROPEEN

(11) **EP 0 408 437 B1**
(45) Date de publication et mention de la délivrance du brevet: **08.06.1994**
(21) Numéro de dépôt: 90401974.2
(22) Date de dépôt: 09.07.1990
(51) Int. Cl.: C07D 277/82, A61K 31/425

(54) **Dérivés d'alkylimino-2 benzothiazoline, leurs procédés de préparation et les médicaments les contenant**
2-Alkyliminobenzothiazoline, ihre Herstellung und sie enthaltende Arzneimittel
2 Alkylimino-benzothiazolines, process for their preparation and pharmaceuticals containing them

(30) Priorité: 13.07.1989 FR 8909481
(43) Date de publication de la demande: 16.01.1991
(73) Titulaire: RHONE-POULENC SANTE, 92160 Antony (FR)
(72) Inventeur: Jimonet, Patrick, F-78450 Villepreux (FR); Nemecek, Conception, F-94600 Choisy Le Roi (FR)
(74) Mandataire: Savina, Jacques

(56) Documents cités:
- EP-A- 0 050 551
- CH-A- 667 091

## Description

La présente invention concerne des dérivés d'alkylimino-2 benzothiazoline de formule : 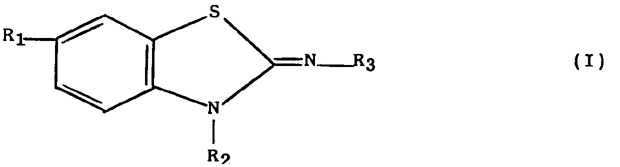 leurs sels, leurs procédés de préparation et les médicaments les contenant. Des dérivés de benzothiazoles sont décrits dans les brevets EP 50551 et CH 667091. Selon l'invention, dans la formule (I),
- R₁ représente un radical polyfluoroalcoxy
- R₂ représente un radical alkylthioalkyle, alkylsulfinylalkyle ou alkylsulfonylalkyle et
- R₃ représente un radical alkyle.

Dans les définitions qui précédent et celles qui seront citées ci-après les radicaux alkyle et les portions alkyle et alcoxy contiennent 1 à 4 atomes de carbone en chaîne droite ou ramifiée.

Les radicaux polyfluoroalcoxy sont de préférence les radicaux trifluorométhoxy, pentafluoroéthoxy, trifluoro -2,2,2 éthoxy ou tétrafluoro -1,1,2,2 éthoxy.

L'invention concerne également les sels d'addition des composés de formule (I) avec les acides minéraux ou organiques.

Selon l'invention, les composés de formule (I) peuvent être préparés par action d'un dérivé de formule : 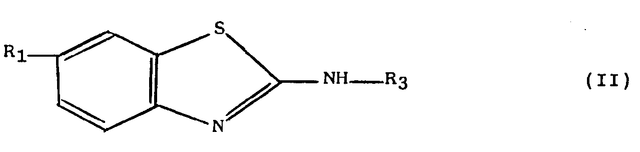 dans laquelle R₁ et R₃ ont les mêmes significations que dans la formule (I), sur un dérivé de formule : R₂ - X (III) dans laquelle R₂ à les mêmes significations que dans la formule (I) et X représente un groupe réactif tel qu'un radical toxyloxy ou un atome d'halogène (de préférence chlore, brome ou iode) ou un sel d'addition d'un tel dérivé avec un acide minéral ou organique.

Cette réaction s'effectue généralement au sein d'un solvant organique inerte tel qu'un alcool (éthanol, propanol...), une cétone (acétone, méthyléthylcétone...) ou le diméthylformamide, à une température comprise entre 20°C et la température d'ébullition du solvant, éventuellement en présence d'iodure de sodium.

Les dérivés de formule (II) peuvent être obtenus par action d'une alkylamine sur un chloro-2 polyfluoroalcoxy-6 benzothiazole.

Cette réaction s'effectue de préférence en milieu aqueux.

Les chloro-2 polyfluoroalcoxy-6 benzothiazole peuvent être obtenus parchloruration d'un hydrazino-2 polyfluoroalcoxy-6 benzothiazole.

La chloruration s'effectue au moyen d'un agent de chloruration, de préférence au moyen de chlorure de thionyle, à une température comprise entre 20°C et 70°C.

Les hydrazino-2 polyfluoroalcoxy-6 benzothiazole peuvent être préparés par action d'hydrazine sur un amino-2 polyfluoroalcoxy-6 benzothiazole.

Cette réaction s'effectue généralement au sein d'un solvant organique tel que l'éthyléneglycol, à une température voisine de 140°C.

Les amino-2 polyfluoroalcoxy-6 benzothiazole peuvent être obtenus par application ou adaptation de la méthode décrite par L.M. YAGUPOL'SKII et coll., Zh. Obshch. Khim., 33(7), 2301(1963).

Les composés de formule (I) pour lesquels R₂ représente un radical alkylsulfinylalkyle ou alkylsulfonylalkyle peuvent également être obtenus par oxydation des dérivés de formule (I) correspondants pour lesquels R₂ représente un radical alkylthioalkyle.

L'oxydation en alkylsulfinylalkyle est effectuée généralement au moyen d'acide m-chloroperbenzoïque, au sein d'un alcool, à une température comprise entre -15°C et -40°C.

L'oxydation en alkylsulfonylalkyle peut s'effectuer au moyen d'eau oxygénée, au sein de l'acide acétique, à une température voisine de 100°C, ou au moyen d'acide m-chloroperbenzoïque au sein d'un solvant inerte tel que le dichlorométhane ou le chloroforme, à une température voisine de 20°C.

Les mélanges réactionnels obtenus par les divers procédés décrits précédemment sont traités suivant des méthodes classiques physiques (évaporation, extraction, distillation, cristallisation, chromatographie...) ou chimiques (formation de sels...).

Les composés de formule (I), sous forme de base libre, peuvent être éventuellement transformés en sels d'addition avec un acide minéral ou organique par action d'un tel acide au sein d'un solvant organique tel qu'un alcool, une cétone, un éther ou un solvant chloré.

Les composés de formule (I) et leurs sels présentent des propriétés pharmacologiques intéressantes. Ces composés sont actifs vis-à-vis des convulsions induites par le glutamate et sont donc utiles dans le traitement et la prévention des phénomènes convulsifs, des troubles schizophréniques et notamment des formes déficitaires de la schizophrénie, des troubles du sommeil, des phénomènes liés à l'ischémie cérébrale ainsi que des affections neurologiques où le glutamate peut être impliqué telles que la maladie d'Alzheimer, la maladie d'Huntington, la sclérose amyotrophique latérale et l'atrophie olivopontocérébelleuse.

L'activité des composés de formule (I) vis-à-vis des convulsions induites par le glutamate a été déterminée selon une technique inspirée de celle de l.P LAPIN, J. Neural. Transmission, vo1.54, 229-238 (1982) ; l'injection du glutamate par voie intra-cérébroventriculaire étant effectuée selon une technique inspirée de celle de R. CHERMAT et P. SIMON, J. Pharmacol. (Paris), vol.6, 489-492 (1975). Leur DE₅₀ est inférieure à 10 mg/kg.

Les composés de formule (I) présentent une toxicité faible. Leur DL₅₀ est supérieure à 15 mg/kg par voie l.P. chez la souris.

Pour l'emploi médicinal, il peut être fait usage des composés de formule (I) tels quels ou à l'état de sels pharmaceutiquement acceptables, c'est-à-dire non toxiques aux doses d'utilisation.

Comme exemples de sels pharmaceutiquement acceptables, peuvent être cités les sels d'addition avec les acides minéraux ou organiques tels que acétate, propionate, succinate, benzoate, fumarate, maléate, oxalate, méthanesulfonate, isothionate, théophylline-acétate, salicylate, phénolphtalinate, méthylène-bis-¡3-oxy- naphtoate, chlorhydrate, sulfate, nitrate et phosphate.

### EXEMPLE 1

Un mélange de 12,4g de méthylamino-2 trifluorométhoxy-6 benzothiazole et de 6,6g de chloro-1 méthylthio-2 éthane dans 75 ml de méthyléthylcétone est chauffé à ébullition pendant 72 heures. Après refroidissement à une température voisine de 20°C, le précipité formé est filtré et lavé par 3 fois 50 ml d'éther éthylique. On obtient 5,7g de chlorhydrate de méthylimino-2 (méthylthio-2 éthyl)-3 trifluorométhoxy-6 benzothiazoline sublimant vers 210°C.

Le méthylamino-2 trifluorométhoxy-6 benzothiazole peut être préparé selon le procédé suivant: 22,0g de chloro-2 trifluorométhoxy-6 benzothiazole et 90 ml de méthylamine aqueuse à 40% sont chauffés en autoclave à 110°C pendant 24 heures. Après refroidissement à une température voisine de 20°C, le milieu réactionnel est ajouté à 300 ml d'eau distillée et le précipité formé filtré et lavé par 2 fois 100 ml d'eau distillée. On obtient après séchage, 19,5g de méthylamino-2 trifluorométhoxy-6 benzothiazole fondant à 162°C.

Le chloro-2 trifluorométhoxy-6 benzothiazole peut être préparé de la manière suivante: A 162 ml de chlorure de thionyle chauffés à 50°C, on ajoute en 1 heure et demie, 133g d'hydrazino-2 trifluorométhoxy-6 benzothiazole. La réaction est poursuivie 1 heure à cette température. Après refroidissement à une température voisine de 20°C, le milieu réactionnel est versé sur 2,5 litres d'eau glacée. Le précipité formé est filtré, lavé avec 2 litres d'eau distillée, puis repris dans 1 litre de dichlorométhane. La phase organique est lavée par 3 fois 200 ml d'eau distillée, puis séchée sur sulfate de magnésium et concentrée à sec sous pression réduite (20 mm de mercure; 2,7kPa). On obtient 122,4g de chloro-2 trifluorométhoxy-6 benzothiazole dont le point de fusion est inférieur à 50°C.

L'hydrazino-2 trifluorométhoxy-6 benzothiazole peut être préparé selon le procédé suivant: Un mélange de 140,4g d'amino-2 trifluorométhoxy-6 benzothiazole, 69,6 ml d'hydrate d'hydrazine à 85% et de 63g de di- chlorhydrate d'hydrazine dans 600 ml d'éthylène glycol est chauffé à 140°C pendant 2 heures sous un courant d'azote. Après refroidissement à une température voisine de 20°C, le précipité formé est filtré, lavé par 4 fois 250 ml d'eau distillée puis par 100 ml d'éther éthylique. On obtient 133,Og d'hydrazino-2 trifluorométhoxy-6 benzothiazole fondant à 208°C.

L'amino-2 trifluorométhoxy-6 benzothiazole peut être préparé selon la méthode décrite par L.M. YAGU-POL'SKII etcoll., Zh. Obshch. Khim., 33(7), 2301(1963).

### EXEMPLE 2

On opère comme à l'exemple 1, à partir de 7,1g de méthylamino-2 trifluorométhoxy-6 benzothiazole et de 4,2g de chloro-1 éthylthio-2 éthane dans 10 ml de méthyléthylcétone. Après 48 heures à ébullition, le milieu réactionnel est refroidi à une température voisine de 20°C. Le précipité formé est filtré et lavé par 3 fois 10 ml de méthyléthylcétone. On obtient, après recristallisation dans 50 ml de propanol-2, 5,3g de chlorhydrate de méthylimino-2 (éthylthio-2 éthyl)-3 trifluorométhoxy-6 benzothiazoline sublimant vers 150°C.

### EXEMPLE 3

A 2,6g de méthylimino-2 (méthylthio-2 éthyl)-3 trifluorométhoxy-6 benzothiazoline en solution dans 40 ml d'éthanol absolu refroidi à -30°C, on ajoute en environ 10 minutes, 2,0g d'acide m-chloroperbenzoïque. La réaction est poursuivie 15 minutes à la même température. Le milieu réactionnel est alors dilué avec 100 ml d'éther éthylique et traité par 2,2 ml d'éther chlorhydrique 4,2N. Le précipité formé est filtré, puis recristallisé dans 30 ml de propanol-2. On obtient 2,Og de chlorhydrate de méthylimino-2 (méthylsulfinyl-2 éthyl)-3 trifluorométhoxy-6 benzothiazoline-(RS) sublimant vers 170°C.

### EXEMPLE 4

A 3,7g de méthylimino-2 (éthylthio-2 éthyl)-3 trifluorométhoxy-6 benzothiazoline en solution dans 50 ml d'éthanol absolu refroidi à -20°C, on ajoute en environ 10 minutes, 2,5g d'acide m-chloroperbenzoïque. La réaction est poursuivie 15 minutes à la même température. Le milieu réactionnel est alors dilué avec 100 ml d'éther éthylique et traité par 2,5 ml d'éther chlorhydrique 4,2N. Le précipité formé est filtré, puis repris dans 50 ml d'eau distillée et neutralisé par de la soude 1N. Après extraction par de l'acétate d'éthyle, séchage sur sulfate de magnésium et concentration à sec sous pression réduite (20 mm de mercure; 2,7kPa), le produit brut est purifié par chromatographie sur colonne de silice avec de l'acétate d'éthyle puis un mélange d'acétate d'éthyle et de méthanol (80-20 en volumes) comme éluants. On obtient, après tranformation en chlorhydrates, 0,5g de chlorhydrate de méthylimino-2 (éthylsulfonyl-2éthyl)-3 trifluorométhoxy-6 benzothiazoline sublimant vers 180°C et 1,9g de méthylimino-2 (éthysulfinyl-2 éthyl)-3 trifluorométhoxy-6 benzothiazoline-(RS) sublimant vers 180°C.

### EXEMPLE 5

Un mélange de 7,45g d'éthylamino-2 trifluorométhoxy-6 benzothiazole et de 3,76g de chloro-1 méthylthio-2 éthane dans 20 ml de méthyléthylcétone est chauffé à ébullition pendant 94 heures. Après refroidissement à une température voisine de 20°C, le précipité formé est filtré et lavé par 2 fois 20 ml de méthyléthyi- cétone. On obtient après recristallisation dans le propanol-2, 0,75g de chlorhydrate d'éthylimino-2 (méthylthio-2 éthyl)-3 trifluorométhoxy-6 benzothiazoline fondant à 208°C. L'éthylamino-2 trifluorométhoxy-6 benzothiazole peut être préparé selon le procédé suivant : 11g de chloro-2 trifluorométhoxy-6 benzothiazole et 80 ml d'éthylamine aqueuse à 33% sont chauffés en autoclave à 110°C pendant 24 heures. Après refroidissement à une température voisine de 20°C, le milieu réactionnel est ajouté à 200 ml d'eau distillée et le précipité formé est filtré et lavé par 2 fois 100 ml d'eau distillée. On obtient 10,32g d'éthylamino-2 trifluorométhoxy-6 benzothiazole fondant à 135°C.

La présente invention concerne également les médicaments constitués par au moins un composé de formule (I) ou un sel d'un tel composé à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, pilules, poudres (capsules de gélatine, cachets ou granulés. Dans ces compositions, le principe actif selon l'invention est mélangé à un ou plusieurs diluants inertes, tels que amidon, cellulose, saccharose, lactose ou silice.

Ces composition peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions non aqueuses, des suspensions ou des émulsions. Comme solvant ou véhicule, on peut employer l'eau, le propy- lèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration asepti- sante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectables qui contiennent, outre le produit actif, des excipients tels que le beurre de cacao, des glycérides semi-synthétiques ou des polyéthylèneglycols.

Les compositions pour administration topique peuvent être par exemple des crèmes, pommades, lotions, collyres, collutoires, gouttes nasales ou aérosols.

En thérapeutique humaine, les composés selon l'invention sont particulièrement utiles dans le traitement et la prévention des phénomènes convulsifs, des troubles schizophréniques et notamment des formes déficitaires de la schizophrénie, des troubles du sommeil, des phénomènes liés à l'ischémie cérébrale et des affections neurologiques où le glutamate peut-être impliqué telles que la maladie d'Alzheimer, la maladie d'Hun- tington, la sclérose amyotrophique latérale et l'atrophie olivopontocérébelleuse.

Les doses dépendent de l'effet recherché, de la durée du traitement et de la voie d'administration utilisée ; elles sont généralement comprises entre 30 et 300mg par jour par voie orale pour un adulte avec des doses unitaires allant de 10 à 100mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

### EXEMPLE A

On prépare, selon la technique habituelle, des gélules dosées à 50mg de produit actif ayant la composition suivante :
- méthylimino-2 [(méthylthio-2 éthyl)-3 trifluorométhoxy-6 benzothiazoline 50 mg
- cellulose 18 mg
- lactose 55 mg
- silice colloïdale 1 mg
- carboxyméthylamidon sodique 10 mg
- talc 10 mg
- stéarate de magnésium 1 mg

### EXEMPLE B

On prépare, selon la technique habituelle, des comprimés dosés à 50mg de produit actif ayant la composition suivante :
- méthylimino-2 (éthylthio-2 éthyl)-3 trifluorométhoxy-6 benzothiazoline 50 mg
- lactose 104 mg
- cellulose 40 mg
- polyvidone 10 mg
- carboxyméthylamidon sodique 22 mg
- talc 10 mg
- stéarate de magnésium 2 mg
- silice colloïdale 2 mg
- mélange d'hydroxyméthylcellulose, glycérine, oxyde de titane (72-3,5-24,5) q.s.p. 1 comprimé pelliculé terminé à 245 mg

### EXEMPLE C

On prépare, une solution injectable contenant 10mg de produit actif avant la composition suivante :
- méthylimino-2 (éthylsulfonyl-2 éthyl)-3 trifluorométhoxy-6 benzothiazoline 10 mg
- acide benzoïque 80 mg
- alcool benzylique 0,06 ml
- benzoate de sodium 80 mg
- éthanol à 95% 0,4 ml
- hydroxyde de sodium 24 mg
- propylène glycol 1,6 ml
- eau q.s.p 4 ml

## Revendications (Revendications pour l'(les) Etat(s) contractant(s) suivant(s) : suivants : AT, BE, CH, DE, DK, FR, GB, IT, LI, LU, NL, SE)

1. Composés de formule : 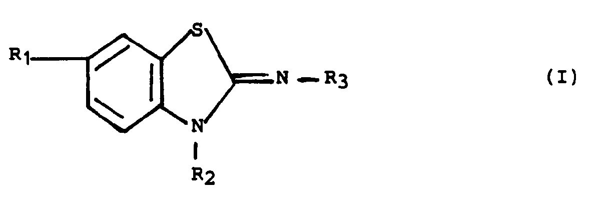 dans laquelle,
- R₁ représente un radical polyfluoroalcoxy
- R₂ représente un radical alkylthioalkyle, alkylsulfinylalkyle ou alkylsulfonylalkyle et
- R₃ représente un radical alkyle
étant entendu que les radicaux alkyle et les portions alkyle et alcoxy contiennent 1 à 4 atomes de carbone en chaîne droite ou ramifiée, ainsi que leurs sels d'addition avec un acide minéral ou organique.

2. Composés selon la revendication 1 pour lesquels R₁ représente un radical trifluorométhoxy, pentafluoroéthoxy, trifluoro-2,2,2 éthoxy ou tétrafluoro-1,1,2,2 éthoxy.

3. Procédé de préparation des composés de formule (I) selon la revendication 1 caractérisé en ce que l'on fait réagir un dérivé de formule : 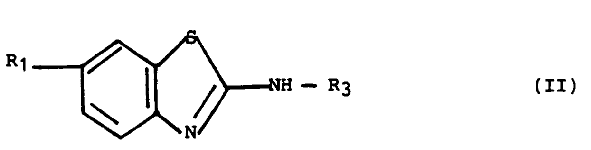 dans laquelle R₁ et R₃ ont les mêmes significations dans la revendication 1, sur un dérivé de formule : 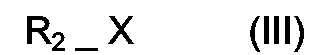 dans laquelle R₂ a les mêmes significations que dans la revendication 1 etX représente un groupe réactif, isole le produit et le transforme éventuellement en sel d'addition avec un acide minéral ou organique.

4. Procédé de préparation des composés de formule (I) selon la revendication 1 pour lesquels R₂ représente un radical alkylsulfinylalkyle ou alkylsulfonylalkyle caractérisé en ce que l'on oxyde un composé correspondant pour lequel R₂ représente un radical alkylthioalkyle, isole le produit et le transforme éventuellement en sel d'addition avec un acide minéral ou organique.

5. Médicaments caractérisés en ce qu'ils contiennent en tant que principe actif Un composé de formule (1) selon la revendication 1 ou un sel d'un tel compose.

## Revendications (Revendications pour l'(les) Etat(s) contractant(s) suivant(s) : suivants : GR, ES)

1. Procédé de préparation des composés de formule : 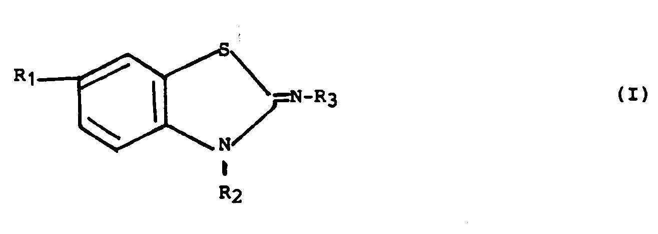 dans laquelle
- R₁ représente un radical polyfluoroalcoxy
- R₂ représente un radical alkylthioalkyle, alkylsulfinylalkyle ou alkylsulfonylalkyle et
- R₃ représente un radical alkyle
étant entendu que les radicaux alkyle et les portions alkyle et alcoxy contiennent 1 à 4 atomes de carbone en chaîne droite ou ramifiée, ainsi que leurs sels d'addition avec un acide minéral ou organique, caractérisé en ce que :
(a) pour la préparation de formule (I) on fait réagir Un dérivé de formule : 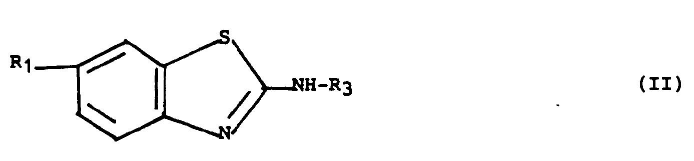 dans laquelle R₁ et R₃ ont les mêmes signif ications que ci-dessus sur un dérivé de formule : R₂-X dans laquelle R₂ a les mêmes signif ications que ci-dessus et x représente un groupe réactif, isole le produit et le transforme éventuellement en sel d'addition avec un acide minéral ou organique.
(b) pour la préparation des composés de formule (I) pour lesquels R₂ représente un radical alkylsulfinylalkyle ou alkylsulfonylalkyle, on oxyde un composé correspondant pour lequel R₂ représente un radical alkylthioalkyle, isole le produit et le transforme éventuellement en sel d'addition avec un acide minéral ou organique.

2. Procédé selon la revendication 1 pour la préparation des composés de formule (I) pour lesquels R₂ représente un radical trifluorométhoxy, pentafluoroéthoxy, trifluoro-2, 2, 2 éthoxy ou tétrafluoro-1, 1, 2, 2 éthoxy.

## Patentansprüche (Patentansprüche für folgende(n) Vertragsstaat(en) : AT, BE, CH, DE, DK, FR, GB, IT, LI, LU, NL, SE)

1. Verbindungen der Formel (I) 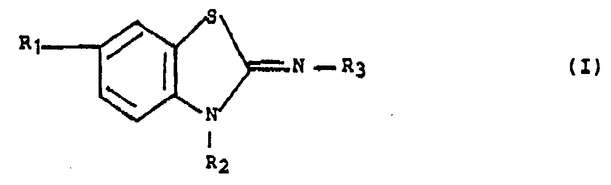 in der
- R₁ einen Polyfluoralkoxy-Rest darstellt,
- R₂ einen Alkylthioalkyl-Rest, Alkylsulfinylalkyl-Rest oder Alkylsulfonylalkyl-Rest bedeutet und
- R₃ ein Alkylrest ist,
mit der Maßgabe, daß die Alkylreste und die Alkyl- und Alkoxyteile 1 bis 4 Kohlenstoffatome in gerader oder verzweigter Kette enthalten, sowie ihre Additionssalze mit einer Mineralsäure oder organischen Säure.

2. Verbindungen nach Anspruch 1, in denen R₁ einen Trifluormethoxy-Rest, einen Pentafluorethoxy-Rest, einen 2,2,2-Trifluorethoxy-Rest oder einen 1,1,2,2-Tetrafluorethoxy-Rest darstellt.

3. Verfahren zur Herstellung der Verbindungen der Formel (I) nach Anspruch 1, dadurch gekennzeichnet, daß man ein Derivat der Formel (ll) 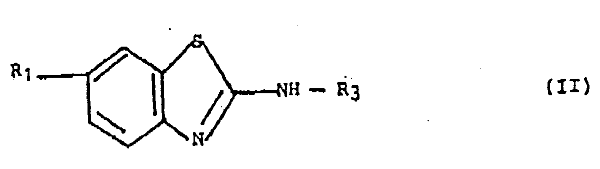 in der R₁ und R₃ die gleichen Bedeutungen wie in Anspruch 1 besitzen, mit einem Derivat der Formel (lll) R₂ - X (lll) zur Reaktion bringt, in der R₂ die gleiche Bedeutung wie in Anspruch 1 besitzt und X eine reaktive Gruppe darstellt, daß man das Produkt isoliert und gegebenenfalls in ein Additionssalz mit einer Mineralsäure oder organischen Säure umwandelt.

4. Verfahren zur Herstellung der Verbindungen der Formel (I) nach Anspruch 1, in der R₂ einen Alkylsulfinylalkyl-Rest oder einen Alkylsulfonylalkyl-Rest darstellt, dadurch gekennzeichnet, daß man eine entsprechende Verbindung, in der R₂ einen Alkylthioalkyl-Rest darstellt, oxidiert, das Produkt isoliert und gegebenenfalls in ein Additionssalz mit einer Mineralsäure oder organischen Säure umwandelt.

5. Medikamente, dadurch gekennzeichnet, daß sie als Wirkstoff eine Verbindung der Formel (I) nach Anspruch 1 oder ein Salz einer derartigen Verbindung enthalten.

## Patentansprüche (Patentansprüche für folgende(n) Vertragsstaat(en) : GR, ES)

1. Verfahren zur Herstellung von Verbindungen der Formel (I) 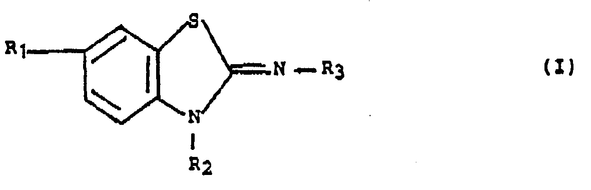 in der
- R₁ einen Polyfluoralkoxy-Rest darstellt,
- R₂ einen Alkylthioalkyl-Rest, Alkylsulfinylalkyl-Rest oder Alkylsulfonylalkyl-Rest bedeutet und
- R₃ ein Alkylrest ist,
mit der Maßgabe, daß die Alkylreste und die Alkyl- und Alkoxyteile 1 bis 4 Kohlenstoffatome in gerader oder verzweigter Kette enthalten, sowie ihren Additionssalzen mit einer Mineralsäure oder organischen Säure.
dadurch gekennzeichnet, daß man
a) zur Herstellung der Verbindungen der Formel (I) ein Derivat der Formel (11) 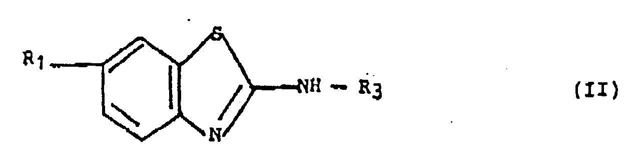 in der R₁ und R₃ die gleichen Bedeutungen wie vorstehend besitzen, mit einem Derivat der Formel R₂-X zur Reaktion bringt, in der R₂ die gleiche Bedeutung wie vorstehend besitzt und X eine reaktive Gruppe darstellt, daß man das Produkt isoliert und gegebenenfalls in ein Additionssalz mit einer Mineralsäure oder organischen Säure umwandelt,
b) zur Herstellung der Verbindungen der Formel (I), in der R₂ einen Alkylsulfinylalkyl-Rest oder einen Alkylsulfonylalkyl-Rest darstellt, eine entsprechende Verbindung, in der R₂ einen Alkylthioalkyl-Rest darstellt, oxidiert, das Produkt isoliert und gegebenenfalls in ein Additionssalz mit einer Mineralsäure oder organischen Säure umwandelt.

2. Verfahren nach Anspruch 1 zur Herstellung der Verbindungen der Formel (I) in der R₁ einen Trifluormethoxy-Rest, einen Pentafluorethoxy-Rest, einen 2,2,2-Trifluorethoxy-Rest oder einen 1,1,2,2-Tetrafluorethoxy-Rest darstellt.

## Claims (Claims for the following Contracting State(s) : AT, BE, CH, DE, DK, FR, GB, IT, Ll, LU, NL, SE)

1. Compounds of formula: 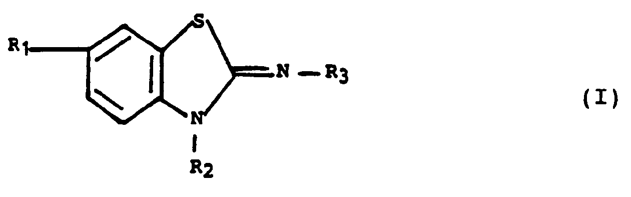 in which
- R₁ represents a polyfluoroalkoxy radical
- R₂ represents an alkylthioalkyl, alkylsulphinylalkyl or alkylsuiphonylalkyl radical and
- R₃ represents an alkyl radical,
it being understood that the alkyl radicals and the alkyl and alkoxy portions contain 1 to 4 carbon atoms in a straight or branched chain, and their addition salts with an inorganic or organic acid.

2. Compounds according to claim 1 for which R₁ represents a trifluoromethoxy, pentafluoroethoxy, 2,2,2-trifluoroethoxy or 1,1,2,2-tetrafiuoroethoxy radical.

3. Process for the preparation of compounds of formula (I) according to claim 1, characterized in that a derivative of formula: 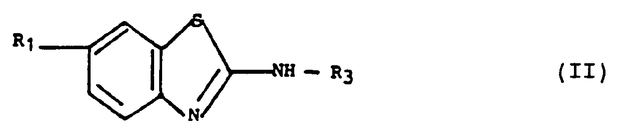 in which R₁ and R₃ have the same meanings as in claim 1 is reacted with a derivative of formula: R₂ - X (III) in which R₂ has the same meanings as in claim 1 and X represents a reactive group, the product is isolated and optionally converted to an addition salt with an inorganic or organic acid.

4. Process for the preparation of the compounds of formula (I) according to claim 1 for which R₂ represents an alkylsulphinylalkyl or alkylsulphonylalkyl radical, characterized in that a corresponding compound for which R₂ represents an alkylthioalkyl radical is oxidized, the product is isolated and optionally converted to an addition salt with an inorganic or organic acid.

5. Medicaments, characterized in that they contain, as active principle, a compound of formula (I) according to claim 1 or a salt of such a compound.

## Claims (Claims for the following Contracting State(s) : GR, ES)

1. Process for the preparation of the compounds of formula: 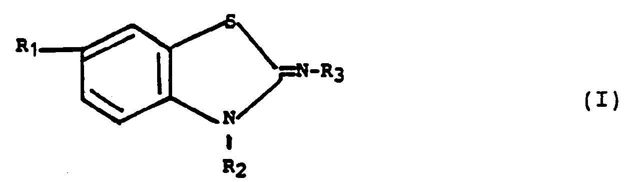 in which
- R₁ represents a polyfluoroalkoxy radical
- R₂ represents an alkylthioalkyl, alkylsulphinylalkyl or alkylsulphonylalkyl radical and
- R₃ represents an alkyl radical,
it being understood that the alkyl radicals and the alkyl and alkoxy portions contain 1 to 4 carbon atoms in a straight or branched chain, and their addition salts with an inorganic or organic acid, characterized in that:
(a) for the preparation of formula (I) a derivative of formula: 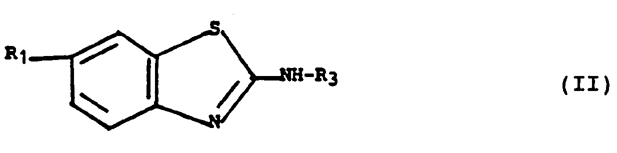 in which R₁ and R₃ have the same meanings as above is reacted with a derivative of formula: R₂-X in which R₂ has the same meanings as above and X represents a reactive group, the product is isolated and optionally converted to an addition salt with an inorganic or organic acid.
(b) for the preparation of the compounds of formula (I) for which R₂ represents an alkylsulphinylalkyl or alkylsulphonylalkyl radical, a corresponding compound for which R₂ represents an alkylthioalkyl radical is oxidized, the product is isolated and optionally converted to an addition salt with an inorganic or organic acid.

2. Process according to claim 1 for the preparation of the compounds of formula (I) for which R₂ represents a trifluoromethoxy, pentafluoroethoxy, 2,2,2-trifluoroethoxy or 1,1,2,2-tetrafluoroethoxy radical.
